# EUROPEAN PATENT APPLICATION

(11) **EP 4 273 265 A1**
(43) Date of publication of application: **08.11.2023**
(21) Application number: 23152305.1
(22) Date of filing: 18.01.2023
(51) Int. Cl.: C12Q 1/686

(54) **BLOODSTREAM INFECTION DETECTION METHODS AND KITS**

(30) Priority: 04.05.2022 US 202263338282 P
(71) Applicant: Precigenome, LLC, San Jose CA 95131 (US); Suzhou Precigenome Ltd. Co., Suzhou City, Jiangsu 215000 (CN)
(72) Inventor: WANG, Yaqi, San Jose, 95131 (US); He, Jing, Suzhou, 215000 (CN); WANG, Zhanying, Suzhou, 215000 (CN); ZHANG, Xuan, Suzhou, 215000 (CN); ZHANG, Hua, Suzhou, 215000 (CN); LING, Yunfeng, San Jose, 95131 (US)
(74) Representative: Wilson Gunn

(57) **Abstract**

Disclosed herein are methods of detecting sepsis in a patient in need thereof, the method comprising: receiving a patient sample, a pathogenic primer mix, a fluorophore label probe, and a dPCR buffer in one or more wells of a microfluidic device and detecting fluorescent light signal from the droplets to determine sepsis in the patient. The microfluidic device comprises a droplet generation channel in fluid communication with the one or more wells, adapted to generate droplets comprising the patient sample, one or more pathogenic primers, a fluorophore label probe, and dPCR buffer. Furthermore, the microfluidic device comprises a chamber in fluid communication with the droplet generation channel for collecting the droplets generated by the droplet generation channel and performing a PCR reaction in the droplets.

## Description

### Priority

This application claims priority to U.S. provisional application with the serial number 63/338,282, filed 05/04/2022, which is incorporated by reference herein in its entirety.

### Field of the Invention

The field of the invention relates to diagnostic devices and methods for the detection of Bloodstream infection.

### Background

All publications herein are incorporated by reference to the same extent as if each individual publication or patent application was specifically and individually indicated to be incorporated by reference. Where a definition or use of a term in an incorporated reference is inconsistent or contrary to the definition of that term provided herein, the definition of that term provided herein applies and the definition of that term in the reference does not apply.

The following description includes information that may be useful in understanding the present invention. It is not an admission that any of the information provided herein is prior art or relevant to the presently claimed invention, or that any publication specifically or implicitly referenced is prior art.

Sepsis is a clinical syndrome of life-threatening organ dysfunction caused by a dysregulated host response due to infection, characterized by high morbidity, disability, and mortality, and is currently one of the causes of in-hospital mortality in patients in intensive care units. Sepsis is a progressive disease in which bloodstream infection (BSI) by pathogens is the main cause of sepsis. The annual number of sepsis patients worldwide exceeds 19 million, of which 6 million die, with a morbidity and mortality rate of more than 1/4, and approximately 3 million of the surviving patients have cognitive impairment. The annual number of sepsis patients in China is estimated at 5.68 million, and the mortality rate of patients with severe sepsis is as high as 33.5%-48.7%. Early recognition and appropriate management can improve the prognosis of patients with sepsis. Whereas the risk of death increases by 58% for every 6 hours of delay in treatment, a two-day delay in treatment will increase the risk of death by 38.8 times. Rapid and accurate identification of causative pathogens is essential for sepsis diagnosis and treatment. Routine blood culture is the gold standard for the diagnosis of bloodstream infection and sepsis, but the blood culture method has two major drawbacks of low detection rate and long detection period. The current blood culture detection rate for sepsis patients is only about 10%, and the detection time takes 48 hours-5 days, which makes it unavoidable to delay the administration of necessary broad-spectrum antibiotics or appropriate antibiotics. Therefore, the establishment of a rapid and accurate method for detecting pathogens infections in the blood of sepsis patients is not only crucial in the diagnosis of sepsis, but also plays a guiding role in the precise treatment. A good prognosis is even less disabling resulting in a burden on the patient, family and society.

Digital PCR (dPCR) is a highly sensitive and direct quantification assay based on the principles of limiting dilution PCR and Poisson statistics. The sample is divided into partitions and there are either zero or one or more target molecules present in any individual reaction. Each partition is separately performed the PCR reaction and analyzed after end-point PCR cycling of the fluorescent signals, and the absolute number of molecules present in the sample is calculated. It does not rely on a standard curve for nucleic acid molecules quantification, which reduces error and improves precision. dPCR can detect down to few copies of viral genomes and has been increasingly applied to infectious diseases. However, there has been no dPCR system that covers or enables multiplex detection of BSI pathogens, which include Gram-negative bacteria, Gram-positive bacteria, fungi, and virus. It is important to efficiently identify them for the successful management of BSI.

Thus, there is still a need for methods, kits, and devices that can accurately and efficiently identify the pathogens associated with sepsis.

### Summary of The Invention

The inventive subject matter provides methods and kits for accurately and efficiently identifying the sepsis pathogens in biological fluid.

In one aspect, the inventive subject matter provides a method of detecting sepsis in a patient in need thereof. The method comprises receiving a patient sample, a pathogenic primer mix, a fluorophore label probe mix, and a dPCR buffer in one or more wells of a microfluidic device and detecting fluorescent light signal from the droplets to determine sepsis in the patient. The microfluidic device comprises a droplet generation channel in fluid communication with the one or more wells, adapted to generate droplets comprising the patient sample, one or more pathogenic primers, one or more fluorophore label probe, and dPCR buffer. Furthermore, the microfluidic device comprises a chamber in fluid communication with the droplet generation channel for collecting the droplets generated by the droplet generation channel and performing a PCR reaction in the droplets.

In preferred embodiments, the pathogenic primer and probe mix comprises Bacteroides fragilis, Enterobacter cloacae, Escherichia coli, Acinetobacter baumannii, Klebsiella pneumoniae, Staphylococcus aureus, Staphylococcus epidermidis, Staphylococcus haemolyticus, Staphylococcus capitis, Staphylococcus hominis, Streptococcus pneumoniae, Pseudomonas aeruginosa, Enterococcus faecalis, Enterococcus faecium, Stenotrophomonas maltophilia, Candida glabrata, Candida parapsilosis, Candida tropicalis, Candida albicans, Proteus mirabilis, Pneumocystis jirovecii, Aspergillus fumigatus, Pichia kudriavzevii, Salmonella enterica, Cryptococcus neoformans, EBV, CMV. The fluorophore label is contemplated to comprise 5' 6-FAM (Fluorescein), Hexachloro-fluorescein (HEX), carboxyrhodamine (ROX), Cyanine 5 (CY5), Cyanine 3 (CY3), JOE, TET, and/or TAMRA.

The microfluidic device disclosed herein preferably comprises multiple wells for receiving the patient sample, primer mix, fluorophore probe, and dPCR buffer. In one embodiment, the microfluidic device comprises at least one well, at least two wells, at least three wells, at least four wells, at least five wells, at least six wells, or at least seven wells. The presence of multiple wells and multiple droplet generation channels enables the simultaneous detection of at least 16 sepsis targets. In this embodiment, a microfluidic device having four wells and four droplet generation chambers is used, and further four pathogenic primer mixes and four fluorescein label probes are added to each of the four wells.

In various embodiments disclosed herein the microfluidic chamber depth is between 50% and 200% of the width or depth of the droplet generation channel such that the collected droplets inside the chamber are arranged in a monolayer fashion.

In one embodiment, the method further comprises an optical detection unit for detecting fluorescent light signal from the droplets, wherein the optical detection unit comprises (a) one or more emission light generators, (b) an optical detector to detect reflected and/or fluoresced light, (c) a chip stage for receiving the microfluidic device, and (d) control and memory circuitry, wherein the control circuitry may move the chip stage in XYZ directions to scan the chamber area in the microfluidic device, and wherein the memory circuitry stores the intensity and wavelength of the reflected and/or fluoresced light detected by the optical detector. Preferably the optical detection unit also comprises an optical reading control unit for optically detecting the light signal from the nucleic acid amplification inside the droplets, counting number of droplets with higher and lower signal, and detecting the size of droplets.

Furthermore, the method disclosed herein may use of a software system for calculating a droplet percentage with lower and higher fluorescent light signal and calculating a size of droplets based on images taken from the optical detection unit. Alternatively, or additionally, there may be a pressure control device for generating droplets in the droplet generation channel.

In preferred embodiments, the methods disclosed herein makes use of a thermal cycling apparatus for conducting nucleic acid amplification in the microfluidic chamber. The hydrodynamic resistance of the chamber is 50 to 1000 times smaller than the hydrodynamic resistance of the droplet generation channel. The hydrodynamic resistance of the chamber is 50 to 100 times smaller than the hydrodynamic resistance of the droplet generation channel. The volume of the chamber is contemplated to be between 20 µL and 500 µL, and the depth of the chamber is between 40 µm to 200 µm. In one embodiment, the depth and the width of the droplet generation channel are each independently between 20 µm to 500 µm. The microfluidic device used in the method disclosed herein is contemplated to comprise between 1 and 8 chambers, each of which in fluid connection with a droplet generation channel.

Various objects, features, aspects and advantages of the inventive subject matter will become more apparent from the following detailed description of preferred embodiments, along with the accompanying drawing figures in which like numerals represent like components.

### Brief Description of The Drawings

Exemplary embodiments are illustrated in referenced figures. It is intended that the embodiments and figures disclosed herein are to be considered illustrative rather than restrictive.
**Fig. 1** depicts, in accordance with embodiments herein, schematic of an integrated microfluidic device. Left: one chamber design. Right: two chambers design. The number of the chambers is not limited, and there may be up to 8 different chambers.
**Fig. 2** depicts, in accordance with embodiments herein, an integrated microfluidic device design.
**Fig. 3** depicts, in accordance with embodiments herein, an integrated microfluidic device design.

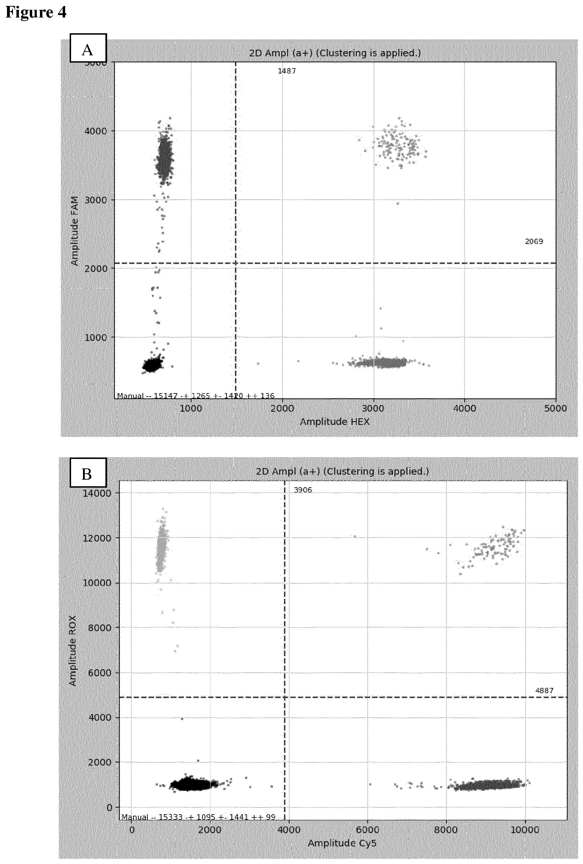

**Fig. 4** depicts, in accordance with embodiments herein, digital PCR graph for panel 1. In Figure 4(A) FAM (green) presents Bacteroides fragilis, HEX (blue) represents Staphylococcus epidermidis. In Figure 4(B) ROX (orange) represents Enterococcus faecium, and Cy5 (Red) represents Streptococcus pneumoniae.

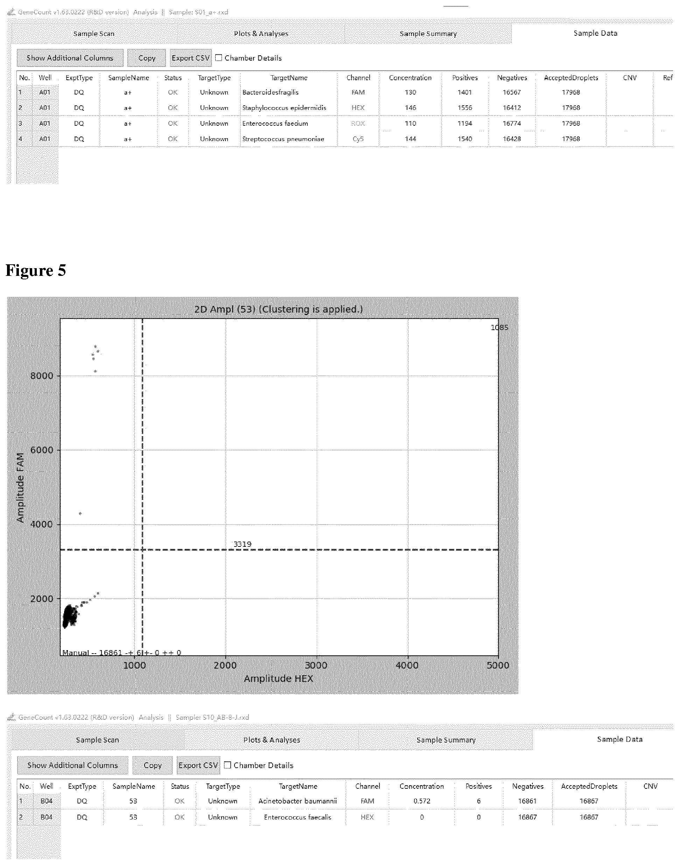

**Fig. 5** depicts, in accordance with embodiments herein, digital PCR graph from a clinical sample. The patient is confirmed with Acinetobacter baumanni infection. Well 1 in Panel 2, FAM channel positive.

### Detailed Description

The present invention relates to a method for detecting a plurality of targets in a biological sample using digital PCR in microfluidic droplets. Preferably, the targets are sepsis targets, for example, sepsis causing bacteria (gram positive and gram negative), virus, and fungi. The targets may also comprise antimicrobial resistant (AMR) genes.

Over 5,000,000 deaths due to sepsis occur globally every year, with over 750,000 hospitalizations and 215,000 deaths in the United States alone. Similar proportions of sepsis deaths occur in other developed countries, such as the United Kingdom. While bacterial infections remain the primary cause of pathogenic sepsis, viruses and fungi comprise a meaningful percentage of sepsis etiologies, especially among immunocompromised patients and those with other comorbidities. As an example, 17% of sepsis as assessed in US hospitals, for example, is attributable to Candida fungal species. Despite varied causes, sepsis detection methods and kits are aimed primarily at bacterial sepsis. First-line treatment often include broad-spectrum antibiotics even before the acquisition of blood cultures. In many cases, treatment may cause more harm than it prevents-antibacterial treatment has no effect on non-bacterial sepsis and is associated with increased mortality when mistakenly administered at high doses to treat fungal sepsis.

The currently disclosed methods, systems, and kits solves the above problem by detecting at least 16 pathogens concurrently, including various bacteria, viruses, fungi, and antimicrobial resistant genes. Thus, the present system provides a simple, accurate, and time-effective detection of sepsis and enables the healthcare provider to decide the treatment steps (antibiotics, viral treatments, antifungal treatments etc).

Accordingly, the inventors have provided a method of using the dPCR system previously disclosed by the inventors in US. Patent No.: US11273444B2, which is incorporated by reference herein in its entirety including the drawings, to quickly and sensitively detect the pathogens causing sepsis.

Importantly, the Limit of Detection (LoD), which is the lowest amount of analyte (for example, microbial or viral or fungal targets) than can be detected, is in the range of 3-400CFU/mL or even lower. In some embodiments, the inventors found that the LoD for the methods disclosed herein is between 50-400 CFU/mL, or more preferably between 50-300 CFU/mL, or more preferably between 50-200 CFU/mL, or more preferably between 50-100 CFU/mL, or more preferably between 25-100 CFU/mL, or more preferably between 10-100 CFU/mL, and more preferably between 5-100 CFU/mL, or more preferably between 3-100 CFU/mL, or more preferably between 3-50 CFU/mL, or more preferably between 3-25 CFU/mL, or more preferably between 3-10 CFU/mL, or most preferably between 3-5 CFU/mL. Thus, because of the lower LoD, the presently disclosed methods enable one to detect the sepsis targets directly from blood samples, without the blood culture steps which usually requires a few hours to grow the sepsis targets. It typically takes 8 hours for the sepsis targets, e.g. S. epidermidis, E. faecalis, E. Coli, E, faecium, A. baumanni, S. pneumoniae, Klebsiella pneumoniae, P. aeruginosa *etc*to grow more than 100folds in concentration. This provides an unexpected advantage over the prior art, which requires blood culture to grow the sepsis bacteria to a range of 5×10³ to 10⁵ CFU/mL before PCR reaction can be performed.

The dPCR system currently includes at least four panels or droplet generation channels to detect 16 pathogenic bacteria including Gram-positive and Gram-negative bacteria. In another embodiment, the inventors have disclosed a kit comprising a microfluidic device having up to seven panels or droplet generation channels to detect at least 17 pathogenic bacteria, 8 fungi, and 2 viruses. In a further embodiment, as further disclosed below, the microfluidic device and methods disclosed herein may detect drug resistance genes in the patient sample thereby allowing the healthcare provider with information on further treatment.

According to a preferred embodiment of the present disclosure, the sepsis causative pathogens include, but are not limited to: Bacteroides fragilis, Enterobacter cloacae, Escherichia coli, Acinetobacter baumannii, Klebsiella pneumoniae, Staphylococcus aureus, Staphylococcus epidermidis, Staphylococcus haemolyticus, Staphylococcus capitis, Staphylococcus hominis, Streptococcus pneumoniae, Pseudomonas aeruginosa, Enterococcus faecalis, Enterococcus faecium,. In another preferred embodiment of the present disclosure, the sepsis causative virus and fungi include, but are not limited to, Candida glabrata, Candida parapsilosis, Candida tropicalis, Candida albicans, Proteus mirabilis, Pneumocystis jirovecii, Aspergillus fumigatus, Pichia kudriavzevii,Salmonella enterica, Cryptococcus neoformans, EBV, CMV.

The drug resistance gene detection kit includes the following antimicrobial drug resistant genes:
i.methicillin resistance- mecA;
ii.vancomycin resistance- vanA, vanB, VanM;
iii.carbapenem resistance- blaKPC, blaGES, blaNDM, blaGIM, OXA-48-like, blaVIM, blaIMP, blaSIM;
iv.Polymyxin resistance- mcr;
v. Tigecycline resistance- acrB, TetX4, rpsJ;
vi.ESBLs- CTX-M-14, CTX-m-15, blaTEM, blaSHV

Preferably, the patient sample containing the sepsis pathogens and/or the antimicrobial drug resistant genes are added to a sample well of a microfluidic device along with a primer mix, a fluorophore probe mix, and a dPCR buffer. This mixture comes in contact with oil that is in a second well and segments the sample to form sample encapsulated into oil droplets (e.g., oil-encapsulated sample droplets).

The microdroplets formed in the droplet generation channel is contemplated to contain a nucleic acid target (for example, DNA from a sepsis causing microorganism in the patient sample) and a heterogeneous mixture of primer pairs and probes, each specific for multiple sites on the nucleic acid target. The microdroplets are also contemplated to contain reagents for conducting a PCR reaction, such as a polymerase and dNTPs.

Primers are single stranded oligonucleotides capable of acting as a point of initiation of DNA synthesis under suitable conditions. Such conditions include those in which synthesis of a primer extension product complementary to a nucleic acid strand is induced in the presence of four different nucleoside triphosphates and an agent for extension (for example, a DNA polymerase or reverse transcriptase) in an appropriate buffer and at a suitable temperature.

The primer is generally composed of single-stranded DNA. They are designed to be complementary to conserved regions of the pathogen (bacteria, virus, fungi) or the drug resistant gene. The appropriate length of a primer depends on the intended use of the primer but typically ranges from about 6 to about 225 nucleotides, including intermediate ranges, such as from 15 to 35 nucleotides, from 18 to 75 nucleotides and from 25 to 150 nucleotides. The design of suitable primers for the amplification of a given target sequence is well known in the art.

In the present disclosure, the pathogenic primer mix comprises DNA primers for several different types of pathogens being detected herein.

The primers and/or probes contemplated herein preferably includes a detectable label. Members of the plurality of probes can each include the same detectable label, or a different detectable label. The detectable label is preferably a fluorescent label. The plurality of probes can include one or more groups of probes at varying concentrations. The one or more groups of probes can include the same detectable label which varies in intensity upon detection, due to the varying probe concentrations.

As a preferred example, the primer can incorporate additional features which allow for the detection or immobilization of the primer but do not alter the basic property of the primer, that of acting as a point of initiation of DNA synthesis. These additional features are known as probes. For example, the probe may be a fluorophore molecule at the 5' end which does not hybridize to the target nucleic acid, but which facilitates detection of the amplified product. The fluorophore probes contemplated herein comprise 5' 6-FAM (Fluorescein), Hexachloro-fluorescein (HEX), carboxyrhodamine(ROX), Cyanine 5 (CY5), Cyanine 3 (CY3), JOE, TET, TAMRA,etc

In one embodiment of the present disclosure, each well of the microfluidic device has the patient sample, 4 primers corresponding to 4 different pathogens, and each probe corresponding to each pathogen having a distinct fluorophore. Thus, in a microfluidic device having 4 wells, this enables the simultaneous detection of 16 pathogens. In this regard, it should be recognized that the patient sample may be a human tissue or body fluid. Exemplary body fluids pus, sputum, semen, urine, blood, plasma, saliva, and cerebrospinal fluid. One example of the arrangement is shown in the table below:

| | FAM | HEX | ROX | CY5 |
|---|---|---|---|---|
| Panel 1 | Bacteroides fragilis | Staphylococcus epidermidis | Enterococcus faecium | Streptococcus pneumoniae |
| Panel 2 | Acinetobacter baumannii | Enterobacter cloacae | Enterococcus faecalis | Staphylococcus aureus |
| Panel 3 | Proteus mirabilis, and DNA extraction & PCR amplification control | Staphylococcus haemolyticus | Klebsiella pneumoniae | Pseudomonas aeruginosa |
| Panel 4 | Escherichia coli | Staphylococcus capitis | Stenotrophomonas maltophilia | Staphylococcus hominis |

| | | | | |
|---|---|---|---|---|
| | | | | |
| | | | | |
| | | | | |
| | | | | |
| | | | | |

The nucleic acid target (for example the sepsis causing pathogen) in each of the formed droplets is amplified, e.g., by thermocycling the droplets under temperatures/conditions sufficient to conduct a PCR reaction. The resulting amplicons in the droplets are then analyzed. For example, the presence of absence of the plurality of targets in the one or more droplets is detected optically, e.g., by the detectable label on the plurality of probes.

In another aspect, disclosed herein is a kit comprising the following kit components:

### Kit Components

| Item No. | Components |
|---|---|
| 1 | Digital PCR buffer |
| 2 | Pathogenic microbes primer probe mix |
| 3 | Positive Control |
| 4 | Negative Control |
| 5 | RNase-/ DNase- H2O |

In some embodiments, the inventors contemplate a microfluidic device kit, wherein the oil well is already loaded with oil and the sample well is loaded with the aforementioned kit components. In this aspect of the inventive subject matter, the user simply loads the patient sample to the sample well, and starts the generation of the droplets followed by dPCR reaction, and fluorescence detection. Preferably, the kit components are in a lyophilized form for ease of transport.

### Microfluidic system and device

The microfluidic system and device contemplated herein comprise appropriate structures to conduct droplet generation, nucleic acid amplification (PCR), and fluorescence detection. The microfluidic device includes a substrate having channels and cross junction for droplet generation, a large chamber for hosting generated droplets and carrying nucleic acid amplification reaction, wells disposed on the substrate for reagent loading, and necessary channels for reagents flowing. The large chamber is positioned between two high resistance channels, where the flow resistances are at least 50 times higher than that of the chamber.

The microfluidic device as disclosed herein integrates droplet generation channels, PCR chamber for droplets, and fluorescence detection. As would be readily apparent to one of skill in the art, while the device and methods disclosed herein illustrate detection by fluorescence, other detection methods, for example, an enzymatic method, or chemiluminescent method may also be used for detection purposes.

In one aspect, the inventors have disclosed herein a microfluidic device, comprising one or more wells for receiving one or more substrates, a droplet generation channel in fluid communication with the one or more wells, wherein the microfluidic channel is adapted to generate droplets, and a chamber in fluid communication with the droplet generation channel, and adapted to collect droplets generated by the droplet generation channel, and further adapted to perform a chemical reaction in the droplets, and further adapted to detect light signal from droplets. The chamber and the droplet generation channel are configured such that the hydrodynamic flow resistance of the chamber is smaller than the hydrodynamic flow resistance of droplet generation channel. The chamber depth is between 50% and 200% of the width or depth of the droplet generation channel such that the collected droplets inside the chamber are arranged in a monolayer fashion. The chamber volume is between 1 to 20 times of water phase volume used for droplet generation.

The droplet may be a water-in oil droplet, or an oil-in-water droplet. A number of chemical reactions are contemplated to be performed in the droplet, for example a DNA amplification reaction, a restriction enzyme digestion, and/or a diagnostic reaction. The microfluidic device may further comprise a pressure control device for generating droplets in the droplet generation channel. The hydrodynamic resistance of the chamber is preferably 50 to 1000 times smaller than the hydrodynamic resistance of droplet generation channel, or more preferably 50 to 100 times smaller than the hydrodynamic resistance of droplet generation channel. The volume of the chamber is between 20 µL and 500 µL. The depth of the chamber is between 40 µm to 500 µm, or in some cases between 20 µm to 200 µm. The depths of channels are between 20 µm to 500 µm, and the width is usually a few centimeters. In some embodiments, a microfluidic channel is used to connect chamber exit to the well. In some embodiments, the device may comprise multiple droplet generation channels and/or multiple chambers, for example between 1 and 8 chambers, each of which are in fluid connection with each other and with a droplet generation channel.

In another aspect, the inventors have disclosed a microfluidic system, comprising the microfluidic device as described above, a thermal cycler comprising a flat surface adapted to receive the microfluidic device, and adapted to raise and lower the temperature of the surface in discrete, pre-programmed steps; and an optical detection unit comprising (a) one or more emission light generators, (b) an optical detector to detect reflected and/or fluoresced light, (c) a chip stage for receiving the microfluidic device, and (d) control and memory circuitry, wherein the control circuitry may move the chip stage in XYZ directions to scan the chamber area in the microfluidic device, and wherein the memory circuitry stores the intensity and wavelength of the reflected and/or fluoresced light detected by the optical detector. The system may further comprise a pressure control device for generating droplets in the droplet generation channel. The optical detection unit optically detects signal from the nucleic acid amplification inside droplets, counting number of droplets with higher and lower signal, and detecting the size of droplets. Furthermore, the microfluidic system may comprise a software system for calculating droplet percentage with lower and higher fluorescent signal, and size of droplets based on images taken from optical unit.

In another aspect, disclosed herein is a method for droplet generation, nucleic acid amplification in droplets, and droplet signal detection in a single microfluidic system, comprising providing a microfluidic system comprising (i) a microfluidic device having one or more wells for receiving one or more substrates; a droplet generation channel in fluid communication with the one or more wells, wherein the microfluidic channel is adapted to generate droplets; and a chamber in fluid communication with the droplet generation channel, and adapted to collect droplets generated by the droplet generation channel; (ii) a thermal cycler comprising a flat surface adapted to receive the microfluidic device, and adapted to raise and lower the temperature of the surface in discrete, pre-programmed steps; and (iii) an optical detection unit comprising one or more emission light generators, an optical detector to detect reflected and/or fluoresced light, a chip stage for receiving the microfluidic device, and control and memory circuitry, wherein the control circuitry may move the chip stage in XYZ directions to scan the chamber area in the microfluidic device, and wherein the memory circuitry stores the intensity and wavelength of the reflected and/or fluoresced light detected by the optical detector; providing a sample comprising a target nucleic acid in a first well, and oil in a second well; segmenting the sample to form sample encapsulated into oil droplets (e.g., oil-encapsulated sample droplets) by providing a continuous flow of sample and oil through the droplet generation channel, and collecting the oil-encapsulated sample droplets in the chamber of the microfluidic device; placing the microfluidic device on a thermal cycler which provides a temperature cycle sufficient to perform nucleic acid amplification in the droplets; and placing the microfluidic device on an optics module for fluorescent detection and quantification of the amplified nucleic acid in the oil encapsulated sample droplet. The chamber and the droplet generation channels are configured such that the hydrodynamic flow resistance of the chamber is smaller than the hydrodynamic flow resistance of droplet generation channel. The chamber depth is between 50% and 200% of the width or depth of the droplet generation channel such that the collected droplets inside the chamber are arranged in a monolayer fashion. The chamber volume is between 1 to 20 times of water phase volume used for droplet generation. The fluorescent detection may comprise orthogonal incident excitation light illumination or inclined incident excitation light illumination. The optics module may detect droplet size information, comprising the steps: scanning the chamber of the microfluidic device to detect the boundary of droplets and calculating the total number of droplets to form droplet boundary information; using the droplet boundary information to identify and locate the droplets; and using the droplet boundary information to find average droplet size, volume, and/or diameter of each of the droplets. The method disclosed herein may further comprise calculating fluorescent signal intensity of each droplet identified and located by the droplet boundary information, or calculating fluorescent signal intensity in each droplet to calculate the fluorescent signal intensity in each droplet, or calculating fluorescent signal intensity in each droplet to calculate the percentage of droplets having positive fluorescence signal, or calculating the concentration of the target polynucleotide by using the estimated average droplets volume based on the boundary information.

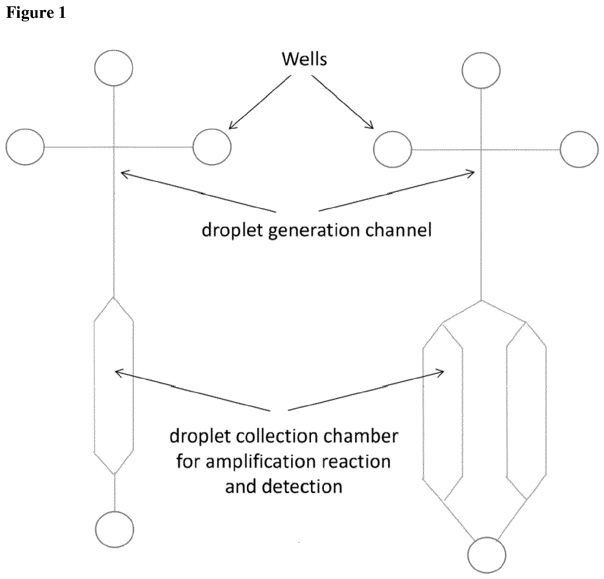

**Fig. 1** is a schematic of an integrated microfluidic device having droplet generation channels, a droplet collection chamber for PCR amplification and fluorescence detection, and wells for reagents loading. In order to generate droplet with diameter in a range of 30 µm-150 µm, the droplet generation channel dimension is in a range of 20 µm-150 µm (width and depth). A chamber is directly connected with droplet generation channel. For some cases, 2 or more chambers can be designed in order to compact the design and hold enough volume of droplets as shown in the right side of **Fig. 1****.** These chambers are in fluid communication with the droplet generation channels. To avoid fluorescence interference among droplets, droplets should be packed inside the chamber in a monolayer fashion. To achieve this monolayer packing, the chamber depth design is based on the droplet size. The depth is in a range of 70%-130% of droplet diameter. Wells are used to hold reagents, for example oil and sample. The chamber volume is the total volume of oil and sample used during droplet generation process.

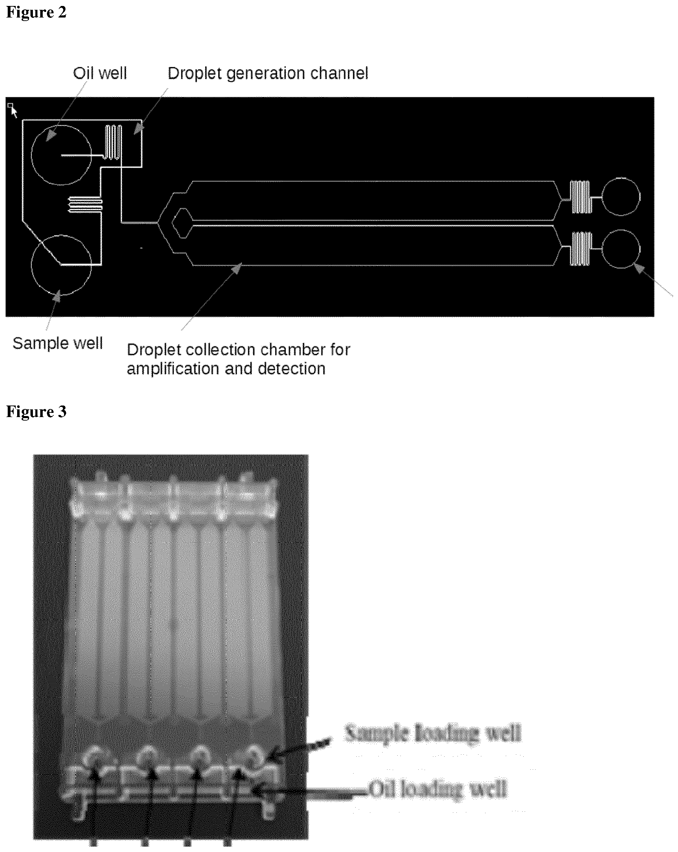

**Fig. 2and 3** illustrates another design of the microfluidic device disclosed herein. In this embodiment, the depth of droplet generation channel is 85 µm, the width is the channel ranges from 70 µm to 90 µm depending on locations. By applying 2-3 psi pressure on oil and sample well, droplets with 110 µm in diameter can be generated. The depth of chamber is 120 µm, in which 110 µm size droplets can form monolayer. The total chamber volume is 40 µL. When 20 µL of sample is used for droplet generation, 20 µL of oil is consumed. In one embodiment, the channels are designed, and the pressure applied to oil and sample phase is set in such a way that the oil to sample consumption ratio is 1:1.

Viewed from a different perspective, the inventors have disclosed a system which is used to achieve droplet generation, droplet collection in chamber, PCR reaction in chamber, and fluorescence detection. The microfluidic device described above sits on top of a thermal cycler which provides temperature cycling. For example, a cycle of 95 °C for 30 seconds (s) and 55 °C for 1 minute inside the chamber for a total of 40 cycles can be achieved using the thermal cycler. A pressure manifold is used to form a good airtight seal. Pressure from 0-20 psi can be applied to wells on the microfluidic chip. To generate droplets in the microfluidic channel, different pressures are used to apply to oil well and sample well. For example, a pressure of 4.5 psi and 4.2 psi were applied to the sample well and oil well respectively to get 110 µm droplet size. After the droplets is collected and packed in the chamber, PCR reaction is started by applying temperature cycle on the film side of microfluidic chip using the thermal cycler. A pressure of 15 psi is applied through the manifold to all the wells on the microfluidic device to reduce evaporation and prevent bubble formation. After PCR reaction, the microfluidic chip device is transferred to a chip stage ofan optics module forbright field and fluorescence detection. The chip stage can be moved in all directions, for example both horizontal and vertical direction. Viewed from another perspective, movement along X, Y, and Z axes are contemplated, such that the optics module can focus the light source inside chamber and scan the whole chamber area. The optics module can scan the droplet chamber to collect sizing information and fluorescent signal in multiple opticalchannels.

The optics module comprises of two main functional sub-modules. One sub-module is used to detect the fluorescent signal of droplets in the chamber. The other sub-module is used to detect the sizing information of the droplets. The optical detection system can be implemented by different approaches.

The fluorescent detection may use orthogonal incident excitation light illumination. To be able to detect multiple fluorescent channels, excitation light, excitation filter, dichroic mirror and emission filter are assembled in to one mechanical structure for one specific fluorescent channel. Multiple assemblies can be installed together in the system, and each time one assembly is switched by a motion control system for that fluorescent channel detection. The excitation light is turned on by a pulsed controlled signal. Simultaneously, the fluorescent signal filtered by the emission filter is collected by the detector. The droplet data can be collected area by area using 2D image scanner or line by line by a line scanner, orthe chip is moved by a XY motion stage for entire chamber scanning.

The fluorescent detection may use inclined incident excitation light illumination. In this implementation, the excited light is shined onto the droplet chamber with a inclined incident angle filtered by a excitation filter. It's also able to detect multiple fluorescent channel signal by switching the excitation light, excitation filter, dichroic mirror and emission filter for specific fluorescent channel, while the detector can be fixed.

Sizing information is used to identify and locate the droplets in the chamber. It is collected by the optical sub-module by dark field or bright field illumination. The droplet size information may be determined using inclined incident light illumination or ring light illumination.

The following discussion provides many example embodiments of the inventive subject matter. Although each embodiment represents a single combination of inventive elements, the inventive subject matter is considered to include all possible combinations of the disclosed elements. Thus, if one embodiment comprises elements A, B, and C, and a second embodiment comprises elements B and D, then the inventive subject matter is also considered to include other remaining combinations of A, B, C, or D, even if not explicitly disclosed.

Embodiments of the present disclosure are further described in the following examples. The examples are merely illustrative and do not in any way limit the scope of the invention as claimed.

### EXAMPLES

### Example: PCR process

In one embodiment, a typical process using the disclosed microfluidic device was described herein. 20µL of sample is loaded in the sample well, and 75µL of oil was loaded in the oil well of the microfluidic device. The chip was loaded in the system. The manifold was lowered, and different pressures (sample: 2.5psi, oil: 2.2psi) are applied to different wells to generate droplets. It typically takes 90s. While generating droplets, the droplets enter the chamberand were packed inside the chamber in a mono layer fashion.

After the droplet generation is done, 15 psi pressure was applied to all wells and the temperature cycle was started. An exemplary temperature cycle for PCR amplification is shown below:

| **dd PCR amplification Program** | | | | |
|---|---|---|---|---|
| **Step** | **Temperature** | **Time** | **heating rate** | **Cycles** |
| Enzyme activation | 95°C | 10 min | 2°C/s | 1 |
| Denaturation | 94°C | 30 sec | | 40 |
| Annealing | 60 °C | 60 Sec | | |
| Enzyme deactivation | 98 °C | 10 min | | 1 |
| Cooling | 20 °C | Hold | | 1 |

After PCR amplification was completed, the microfluidic chip was transferred to the second stage. Bright field and fluorescence images were taken by the system. Data analysis was performed by software to give DNA concentration. As would be readily apparent to one of skill in the art, other temperatures, pressure, and times may be used as well to practice the method disclosed herein.

### Specimen Requirements

### Applicable specimen types: whole blood, serum, plasma

Specimen storage and transportation: EDTA anticoagulant is recommended, heparin anticoagulant should not be used. Specimens can be immediately processed or stored for up to 48 hours if stored at 2-8°C during transportation. If specimens cannot be tested within 48 hours should be stored at -20°C or below. Multiple freeze-thaw cycles should be avoided. Specimens should be transported in a sealed frozen pitcher with ice/ice bags or in a sealed foam box with ice/ice bags.

### Specimen processing requirements

Blood sample: peripheral blood sample shall not be less than 2ml, and 4 ml is recommended to improve the detection rate. Both plasma and whole blood can be used in the methods disclosed herein. If plasma is used, it is recommended to separate plasma within 2 hours after blood was drawn, generally not more than 4 hours, and the plasma volume shall not be less than 2ml (4ml is recommended). Centrifuge at 800 rpm for 10 min and take the supernatant. If plasma cannot be separated in time, vacuum blood collection vessels can be used for room temperature storage, or special normal temperature blood collection vessels (streck tubes) containing free DNA protective agents and anti-cell lysis protective agents can be used for storage for no more than 3 days. If whole blood is used, it is recommended to break it first by physical method. It is recommended to use a commercial kit to extract microbial DNA from blood samples. After DNA extraction, it is recommended to perform the test immediately, otherwise please store it below - 20 °C for a period of no more than 3 months.

### Test procedure

Prepare (14 +4*n, 4 tubes of positive control, 4 tubes of negative control, 4*n tubes of sample to be tested) tubes of PCR amplification reagent mix according to the table below, and vortex and mix well, centrifuge instantly.

| Reagent | Volume (µL) |
|---|---|
| Digital PCR Premix (Probe Method) | 5 |
| Panel 1 / Panel 2 / Panel 3 / Panel 4 Primer Probe Mix | 1 |
| Sample (template)^{∗} or Positive QC or Negative QC** | Variable |
| RNase-/ DNase-water | Add to 20µL |
| Total | 20 |

| | |
|---|---|
| *For microbial DNA, it is recommended to concentrate the nucleic acids extracted from the same sample in a volume of 100 µl if possible **Positive controls are added in 4µL volume; Negative controls are added in 4µL volume. | |

### Micro-droplet generation and amplification

Add 75µl of droplet generating oil to the oil well in the high-throughput digital PCR chip, add 20µl of amplification mix to the reagent well, then add 10µl of sealing oil on the top of amplification mix, cover with the sealing lids, and place on the PCR amplification instrument (SG-2000).

The layout of chip placement showed in the following recommended order.

| **Chip 1** | | | |
|---|---|---|---|
| Hole 1 | Hole 2 | Hole 3 | Hole 4 |
| Panel 1-PC | Panel 2-PC | Panel 3-PC | Panel 4-PC |

| **Chip 2** | | | |
|---|---|---|---|
| Hole 1 | Hole 2 | Hole 3 | Hole 4 |
| Panel 1-Negative control | Panel 2-Negative control | Panel 3-Negative control | Panel 4-Negative control |

| **Chip 3** | | | |
|---|---|---|---|
| Hole 1 | Hole 2 | Hole 3 | Hole 4 |
| Panel 1-samlpe 1 | Panel 2-samlpe 1 | Panel 3-samlpe 1 | Panel 4-samlpe 1 |

| **Chip 4** | | | |
|---|---|---|---|
| Hole 1 | Hole 2 | Hole 3 | Hole 4 |
| Panel 1-samlpe 2 | Panel 2-samlpe 2 | Panel 3-samlpe 2 | Panel 4-samlpe 2 |

The amplification reaction is carried out according to the following recommended SG-2000 amplification procedure:

| | **dd-PCRprocedure** | | | |
|---|---|---|---|---|
| Steps | | Temperature | Time | Cycles |
| 1 | Enzyme activation | 95°C | 10min | 1 |
| 2 | Denature | 94°C | 30sec | 40 |
| | Annealing/ Extension | 60°C | 1 min | |
| 3 | Inactivation | 98°C | 10 min | 1 |
| 4 | Cooling | 20°C | Hold | 1 |

### Signal collection

Remove the high-throughput digital PCR chip from the PCR amplifier (SG-2000) and horizontally transfer it to the biochip reader for signal collection. Select DQ mode on the biochip reader, fluorescence channel settings FAM, HEX, ROX, and Cy5.

### Result Analysis

The experimental data are analyzed on the Biochip Reader. The accepted droplets should be greater than 10,000 for subsequent analysis. A 2D scatter plot of each of channel 1, channel 2, channel 3, and channel 4 is viewed. The black dots represent droplets without amplification template, green dots represent droplets containing FAM-labeled probe, blue dots represent droplets containing HEX-labeled probe, orange dots represent droplets containing ROX-labeled probe, fuchsia dots represent droplets containing CY5 probe, and red dots represent droplets containing both sets of probes.

### Interpretation of test results

Reference range Detection of 16 pathogenic microorganisms, including Gram-positive and Gram-negative bacteria, which exist in infected patients' blood stream.

Interpretation of test results Laboratory environment pollution, reagent contamination, and specimen cross-contamination will cause false positive results; Improper reagent transportation, storage, or inaccurate reagent preparation may result in a decline in the reagent detection efficiency, false negatives or inaccurate quantitative detection. Positive droplets between 1-3 indicate that the specimen concentration is too low, or there are interfering substances that inhibit the reaction.

### Limitation of the test method

Test results are affected by how the specimen is collected, processed, transported and stored. Loss of control in any of the steps will lead to incorrect results. Special attention should be paid to the risk of specimen cross contamination during processing which may lead to false positive results.

Improper specimen collection, transport and processing may lead to false negative results; mutation of target sequence may lead to false positive or false negative results.

This kit is applicable to specified specimen types and detection system, including digital PCR instruments, nucleic acid extraction reagents, detection method, etc. Validation is required before applying any new specimen types or detection system.

The above test reagent is applicable to the detection of the listed 28 pathogenic bacteria,fungi, and virus, excluding the detection of microorganisms other than those stated. However, it should be noted that the presently disclosed method can be used to detect up to 36 pathogens, or up to 48 pathogens, or up to 64 pathogens

### Laboratory Quality Control

At least 4 sets of positive quality control reactions containing DNA from the 16 microorganism strains to be tested should be included.

### At least 4 sets of negative control samples should be included

Refer to the data for positive and negative QC reactions to delineate the threshold for the positive and negative droplets. Place a threshold by using the median line between negative droplets and positive droplets of the Positive QC. For example, FAM>3000 fluorescence intensity values for panel 1 in positive QC are FAM positive droplets, FAM negative QC droplets fluorescence intensity < 1000; HEX>4000 fluorescence intensity values are HEX positive droplets, HEX negative QC droplets fluorescence intensity < 1000, ROX>7500 fluorescence intensity values are ROX positive droplets, ROX negative QC droplets fluorescence intensity < 1000, Cy5>2000 fluorescence intensity value for Cy5 positive droplets, Cy5 negative QC droplets fluorescence intensity < 1000, samples to be tested can be referred to FAM between 500-4000, HEX between 1000-4000, ROX between 1000-7500, Cy5 between 1000-2500 to set the threshold line in differentiating the panel 1 assayed negative/positive droplets.

### Interpretation of positive samples:

| **Bacteria** | **Positive Droplets** | **Interpretation** |
|---|---|---|
| *Bacteroides fragilis* | 0-2 positive droplets | Negative |
| | ≥ 3 positive droplets | Positive |
| | 0-2 positive droplets | Negative |
| *Staphylococcus epidermidis* | ≥ 3 positive droplets | Positive |
| *Enterococcus faecium* | 0-2 positive droplets | Negative |
| | ≥ 3 positive droplets | Positive |
| *Streptococcus pneumoniae* | 0-2 positive droplets | Negative |
| | ≥ 3 positive droplets | Positive |
| *Acinetobacter baumannii* | 0-2 positive droplets | Negative |
| | ≥ 3 positive droplets | Positive |
| *Enterobacter cloacae* | 0-2 positive droplets | Negative |
| | ≥ 3 positive droplets | Positive |
| *Enterococcus faecalis* | 0-2 positive droplets | Negative |
| | ≥ 3 positive droplets | Positive |
| *Staphylococcus aureus* | 0-2 positive droplets | Negative |
| | ≥ 3 positive droplets | Positive |
| *Staphylococcus haemolyticus* | 0-2 positive droplets | Negative |
| | ≥ 3 positive droplets | Positive |
| *Klebsiella pneumoniae* | 0-2 positive droplets | Negative |
| | ≥ 3 positive droplets | Positive |
| *Pseudomonas aeruginosa* | 0-3 positive droplets | Negative |
| | ≥ 4 positive droplets | Positive |
| *Escherichia coli* | 0-2 positive droplets | Negative |
| | ≥ 3 positive droplets | Positive |
| *Staphylococcus capitis* | 0-2 positive droplets | Negative |
| | ≥ 3 positive droplets | Positive |
| *Stenotrophomonas maltophilia* | 0-4 positive droplets | Negative |
| | ≥ 5 positive droplets | Positive |
| *Staphylococcus hominis* | 0-2 positive droplets | Negative |
| | ≥ 3 positive droplets | Positive |
| *Proteus mirabilis* | 0-2 positive droplets | Negative |
| | ≥ 3 positive droplets | Positive |

Negative QC is particularly important when testing samples with very low numbers of microbe infections. For negative QC, the number of positive droplets in each fluorescent channel should theoretically be 0. If there are positive droplets in each fluorescent channel or in one fluorescent channel in negative QC without DNA template and negative QC, it is recommended to check whether the laboratory is contaminated with aerosols. To ensure the detection effect, it is recommended to change the laboratory, replace the reaction water, pipettor and tips, PCR tubes, and other experimental equipment, and reformulate the reaction solution.

Invalid result interpretation: the total number of droplets in each reaction well should be >10000. if it is lower than 10000, the micro-droplet generation in that reaction well is not satisfactory and the sample in that well should be retested again.

Invalid result interpretation: A negative result for the DNA extraction and PCR amplification control gene of the FAM channel of panel 3 indicates that there is a problem in the DNA extraction and digital PCR amplification and the whole panels should be retested again.

If there are "raindrops" in the middle of negative and positive droplets, the threshold value should be further determined according to the fluorescence values of positive QC and negative QC.

In one embodiment, the original strain stock solution is diluted according to the ratio in the table. Diluted DNA template was added according to the volume in the table, and mixed with water to the total sample volume of 8 µL. The total volume of the PCR mix added to the sample well on the microfluidic cartridge was 20 µL. LoD of the digital PCR assay was determined by the concentration in CFU/mL of the lowest dilution that can be detected, described in Table 1.

**Table 1:LoD detected using digital PCR assay described in this invention.**

| | Target Strain | Strain Stock solution concentration (CFU/µL) | Dilution factor | Concentration after dilution (CFU/µL) | DNA template input volume/µL (Total Sample volume is 8 µL) | LOD (8 µl) CFU/reaction | LoD* (CFU/ml) |
|---|---|---|---|---|---|---|---|
| Well 1 | Bacteroides fragilis (BF) | 2.30E+06 | 25518.02 | 90.13 | 0.32 | 28.84 | 180.26 |
| | Staphylococcus epidermidis (EPI) | 2. 30E+06 | 25518.02 | 90.13 | 0.32 | 28.84 | 180.26 |
| | Enterococcus faecalis (EFM) | 1.50E+06 | 498007.97 | 3.01 | 0.48 | 1.45 | 9.04 |
| | Streptococcus pneumoniae (SP) | 1.00E+07 | 39834.29 | 251.04 | 0.32 | 80.33 | 502.08 |
| Well 2 | Acinetobacter baumannii (AB) | 2. 10E+06 | 413223.14 | 5.08 | 0.427 | 2.17 | 13.56 |
| | Enterobacter cloacae (EC) | 2. 10E+06 | 162127.11 | 12.95 | 0.32 | 4.14 | 25.91 |
| | Enterococcus faecium (EFS) | 1.30E+06 | 213128.73 | 6.10 | 0.32 | 1.95 | 12.20 |
| | Staphylococcus aureus (SA) | 2.40E+06 | 41044.16 | 58.47 | 0.32 | 18.71 | 116.95 |
| Well 3 | Proteus mirabilis (PM) | 2. 10E+06 | 162127.11 | 12.95 | 0.32 | 4.14 | 25.91 |
| | Staphylococcus haemolyticus (HAE) | 1.20E+06 | 25378.13 | 47.28 | 0.32 | 15.13 | 94.57 |
| | Klebsiella pneumoniae (KP) | 1.90E+06 | 348189.42 | 5.46 | 0.32 | 1.75 | 10.91 |
| | Pseudomonas aeruginosa(PA) | 2.80E+06 | 63548.55 | 44.06 | 0.32 | 14.10 | 88.12 |
| Well 4 | Escherichia coli (ECO) | 2. 30E+06 | 228519.20 | 10.06 | 0.32 | 3.22 | 20.13 |
| | Staphylococcus cephalosporus (CAP) | 2.60E+06 | 64135.45 | 40.54 | 0.32 | 12.97 | 81.08 |
| | Stenotrophomonas maltophilia (SM) | 1.50E+06 | 38092.34 | 39.38 | 0.32 | 12.60 | 78.76 |
| | Staphylococcus hominis (HOM) | 1.60E+06 | 33579.58 | 47.65 | 0.32 | 15.25 | 95.30 |

| | | | | | | | |
|---|---|---|---|---|---|---|---|
| *LoD is back calculated based on 2 mL original sample solution extracted and eluted in 100 µL elution buffer. | | | | | | | |

In some embodiments, the numbers expressing quantities of ingredients, properties such as concentration, reaction conditions, and so forth, used to describe and claim certain embodiments of the invention are to be understood as being modified in some instances by the term "about." Accordingly, in some embodiments, the numerical parameters set forth in the written description and attached claims are approximations that can vary depending upon the desired properties sought to be obtained by a particular embodiment. In some embodiments, the numerical parameters should be construed in light of the number of reported significant digits and by applying ordinary rounding techniques. Notwithstanding that the numerical ranges and parameters setting forth the broad scope of some embodiments of the invention are approximations, the numerical values set forth in the specific examples are reported as precisely as practicable. The numerical values presented in some embodiments of the invention may contain certain errors necessarily resulting from the standard deviation found in their respective testing measurements.

Unless the context dictates the contrary, all ranges set forth herein should be interpreted as being inclusive of their endpoints and open-ended ranges should be interpreted to include only commercially practical values. Similarly, all lists of values should be considered as inclusive of intermediate values unless the context indicates the contrary.

As used in the description herein and throughout the claims that follow, the meaning of "a," "an," and "the" includes plural reference unless the context clearly dictates otherwise. Also, as used in the description herein, the meaning of "in" includes "in" and "on" unless the context clearly dictates otherwise.

The recitation of ranges of values herein is merely intended to serve as a shorthand method of referring individually to each separate value falling within the range. Unless otherwise indicated herein, each individual value with a range is incorporated into the specification as if it were individually recited herein. All methods described herein can be performed in any suitable order unless otherwise indicated herein or otherwise clearly contradicted by context. The use of any and all examples, or exemplary language (e.g. "such as") provided with respect to certain embodiments herein is intended merely to better illuminate the invention and does not pose a limitation on the scope of the invention otherwise claimed. No language in the specification should be construed as indicating any non-claimed element essential to the practice of the invention.

Groupings of alternative elements or embodiments of the invention disclosed herein are not to be construed as limitations. Each group member can be referred to and claimed individually or in any combination with other members of the group or other elements found herein. One or more members of a group can be included in, or deleted from, a group for reasons of convenience and/or patentability. When any such inclusion or deletion occurs, the specification is herein deemed to contain the group as modified thus fulfilling the written description of all Markush groups used in the appended claims.

It should be apparent to those skilled in the art that many more modifications besides those already described are possible without departing from the inventive concepts herein. The inventive subject matter, therefore, is not to be restricted except in the spirit of the appended claims. Moreover, in interpreting both the specification and the claims, all terms should be interpreted in the broadest possible manner consistent with the context. In particular, the terms "comprises" and "comprising" should be interpreted as referring to elements, components, or steps in a non-exclusive manner, indicating that the referenced elements, components, or steps may be present, or utilized, or combined with other elements, components, or steps that are not expressly referenced. Where the specification claims refers to at least one of something selected from the group consisting of A, B, C ... and N, the text should be interpreted as requiring only one element from the group, not A plus N, or B plus N, etc.

## Claims

1. A method of detecting bloodstream infection in a patient in need thereof, the method comprising:
receiving a patient sample, a pathogenic primer mix, a fluorophore label probe, and a dPCR buffer in one or more wells of a microfluidic device;
wherein the microfluidic device comprises a droplet generation channel in fluid communication with the one or more wells, adapted to generate droplets comprising the patient sample, one or more pathogenic primers, the fluorophore label probe, and dPCR buffer;
wherein the microfluidic device comprises a chamber in fluid communication with the droplet generation channel for collecting the droplets generated by the droplet generation channel and performing a PCR reaction in the droplets;
detecting fluorescent light signal from the droplets to determine bloodstream infection in the patient.

2. The method of claim 1, wherein the bloodstream infection is sepsis, and/or wherein the sepsis causing pathogens comprise at least about 3-400 colony forming units per mL (CFU/mL).

3. The method of any one of the preceding claims, wherein the patient sample comprises sepsis causing pathogens.

4. The method of any one of the preceding claims, wherein the pathogenic primer mix comprises Bacteroides fragilis, Enterobacter cloacae, Escherichia coli, Acinetobacter baumannii, Klebsiella pneumoniae, Staphylococcus aureus, Staphylococcus epidermidis, Staphylococcus haemolyticus, Staphylococcus capitis, Staphylococcus hominis, Streptococcus pneumoniae, Pseudomonas aeruginosa, Enterococcus faecalis, Enterococcus faecium, Stenotrophomonas maltophilia, Candida glabrata, Candida parapsilosis, Candida tropicalis, Candida albicans, Proteus mirabilis, Pneumocystis jirovecii, Aspergillus fumigatus, Pichia kudriavzevii,Salmonella enterica, Cryptococcus neoformans, EBV, and/or CMV.

5. The method of any one of the preceding claims, wherein the fluorophore label comprises 5' 6-FAM (Fluorescein), Hexachloro-fluorescein (HEX), carboxyrhodamine (ROX), Cyanine 5 (CY5), Cyanine 3 (CY3), JOE, TET, and/or TAMRA.

6. The method of any one of the preceding claims, wherein the microfluidic device comprises at least one well, at least two wells, at least three wells, at least four wells, at least five wells, at least six wells, or at least seven wells.

7. The method of any one of the preceding claims, further comprising simultaneous detection of at least 16 sepsis targets, comprising a microfluidic device having four wells and four droplet generation chambers, and further comprising adding four pathogenic primer mixes and four fluorescein label probes in each of the four wells.

8. The method of any one of the preceding claims, wherein the chamber depth is between 50% and 200% of the width or depth of the droplet generation channel such that the collected droplets inside the chamber are arranged in a monolayer fashion.

9. The method of any one of the preceding claims, further comprising an optical detection unit for detecting fluorescent light signal from the droplets, wherein the optical detection unit comprises (a) one or more emission light generators, (b) an optical detector to detect reflected and/or fluoresced light, (c) a chip stage for receiving the microfluidic device, and (d) control and memory circuitry, wherein the control circuitry may move the chip stage in XYZ directions to scan the chamber area in the microfluidic device, and wherein the memory circuitry stores the intensity and wavelength of the reflected and/or fluoresced light detected by the optical detector.

10. The method of claim 9, further comprising an optical reading control unit for optically detecting the light signal from the nucleic acid amplification inside the droplets, counting number of droplets with higher and lower signal, and detecting the size of droplets.

11. The method of any one of the preceding claims, further comprising a software system for calculating a droplet percentage with lower and higher fluorescent light signal and calculating a size of droplets based on images taken from the optical detection unit.

12. The method of any one of the preceding claims, further comprising a pressure control device for generating droplets in the droplet generation channel.

13. The method of any one of the preceding claims, further comprising a thermal cycling apparatus for conducting nucleic acid amplification in the chamber.

14. The method of any one of the preceding claims, wherein the hydrodynamic resistance of the chamber is 50 to 1000 times smaller than the hydrodynamic resistance of the droplet generation channel.

15. The method of any one of the preceding claims, wherein the volume of the chamber is between 20 µL and 500 µL, and/or wherein the depth of the chamber is between 40 µm to 200 µm, and/or wherein the depth and the width of the droplet generation channel are each independently between 20 µm to 500 µm.
